# EUROPÄISCHE PATENTSCHRIFT

(11) **EP 0 846 469 B1**
(45) Veröffentlichungstag und Bekanntmachung des Hinweises auf die Patenterteilung: **15.01.2003**
(21) Anmeldenummer: 97119490.7
(22) Anmeldetag: 07.11.1997
(51) Int. Cl.: A61M 1/00, A61M 1/36, B01D 19/00

(54) **Blutabsaugvorrichtung**
Blood suction device
Appareil d'aspiration du sang

(30) Priorität: 05.12.1996 DE 19650406
(43) Veröffentlichungstag der Anmeldung: 10.06.1998
(73) Patentinhaber: Convergenza AG, 9490 Vaduz (LI)
(72) Erfinder: Brockhoff, Alexander, 9494 Schaan (LI); Plechinger, Hans, Cranbrook, B.C. VIC 6J5, CANADA (CA)
(74) Vertreter: Vetter, Ewald Otto, Dipl.-Ing.

(56) Entgegenhaltungen:
- EP-A- 0 318 993
- EP-A- 0 778 031
- DE-A- 2 611 383
- DE-A- 4 326 886
- US-A- 4 710 299

## Beschreibung

Die Erfindung betrifft eine Blutabsaugvorrichtung zum Absaugen von Blut von einem Patienten.

Eine Blutabsaugvorrichtung vergleichbarer Art ist beispielsweise aus der DE-A-4 326 886 bekannt.

Wenn von einem Patienten Blut abgesaugt wird, insbesondere von einer Wundstelle des Patienten, welche durch eine Operation oder einen Unfall entstehen kann, und dieses Blut anschließend für den gleichen Patienten oder einen anderen Patienten wieder verwendet werden soll, dann muß das Blut möglichst sofort und möglichst nahe bei der Absaugstelle des Patienten wieder von der Luft getrennt werden, welche gegebenenfalls an der Absaugstelle des Patienten in das Blut eingesaugt wurde. Einem Patienten darf kein Blut zugeführt werden, welches Luft enthält. Außerdem wird das Blut durch Lufteinschlüsse geschädigt, je länger eingesaugte Luft im Blut verbleibt und je stärker die Luft auf dem Blutabsaugweg mit diesem Blut vermischt wird.

Die Qualität von Blut kann unter anderem beeinträchtigt werden: durch die Menge und die Zeitdauer von Luft oder anderen Gasen im Blut; durch Saug- oder Druckkräfte auf das Blut; durch Reibungskräfte des Blutes in Strömungswegen; Umlenkungen der Blutströmung und Turbulenzen im Blut.

Beim Entgasen von Blut müssen verschiedene Anwendungsfälle unterschieden werden, da sie verschiedene Aufgaben und verschiedene Bedingungen haben:

**1. Anwendungsfall:** Das Entgasen von Blut, während es in verhältnismäßig kleinen und stark sich ändernden Mengen von einem Patienten abgesaugt wird, insbesondere durch eine Saugkanüle von einer Wundstelle des Patienten, welche durch einen Unfall oder während einer Operation entstehen kann. Der Unterdruck zum Absaugen des Blutes wird durch eine Saugpumpe, meistens eine Rollerpumpe, erzeugt. Die Saugpumpe saugt nicht nur Blut, sondern an der Blutabsaugstelle des Patienten häufig auch Luft ab. Der abgesaugte Luftanteil ist verhältnismäßig hoch zu dem abgesaugten Blutanteil, beispielsweise 5 Teile Luft zu einem Teil Blut (Volumenteile). Die Blutabsaugrate ist verhältnismäßig niedrig, beispielsweise 100 bis 600 Milliliter pro Minute, und schwankt stark. Die Absaug-Strömungsgeschwindigkeit des Blutes ist ebenfalls verhältnismäßig niedrig und schwankt stark.

Die im Blut enthaltenen Luftbläschen sind häufig verhältnismäßig groß. Ihre Größe reicht vom Mikrometerbereich bis zum Millimeterbereich. Die vorliegende Erfindung betrifft diesen Anwendungsbereich, nämlich das Entgasen von Blut, während es von einem Patienten abgesaugt wird.

**2. Anwendungsfall:** Das Zuführen von Blut zu einem Patienten, beispielsweise bei der Blutwäsche (Dialyse) oder bei hohem Blutverlust bei einem Unfall. Blut, welches einem Patienten zugeführt wird, kann nicht gesaugt werden, sondern kann nur durch Druck einer Druckpumpe dem Patienten zugeführt werden. Die Blutrate, beispielsweise 3 Liter pro Minute, und die Fördergeschwindigkeit des Blutes sind verhältnismäßig hoch und im wesentlichen konstant. Der Luftanteil im Blut ist jedoch gering, beispielsweise nur 50 x 10⁻⁶ Volumenteile Luft zu einem Volumenteil Blut. Die Luft hat die Form von nur sehr kleinen Bläschen im Mikrometerbereich.

**3. Anwendungsfall:** Die Behandlung von Blut außerhalb und unabhängig von einem Patienten. Hier hat man ähnlich wie beim 2. Anwendungsfall im wesentlichen konstante Blutströmungsmengen und konstante Blutströmungsgeschwindigkeiten.

Zum ersten Anwendungsfall ist aus der US-A-3 785 380 eine Blutabsaugvorrichtung bekannt, welche aus einem zylindrischen Gehäuse, in welchem sich mikroporöses Filtermaterial zum Ausfiltern von Luftbläschen und anderen Verunreinigungen aus einem von einem Patienten abgesaugten Blutstrom befinden, einer Blutabsaugkanüle an einem vorderen Ende des Gehäuses und aus einer Blutabsaugleitung am hinteren Ende des Gehäuses besteht.

Zum genannten zweiten Anwendungsfall ist folgende Literatur bekannt: GB-A-2 063 108 zeigt eine Blutentgasungsvorrichtung zum Entfernen von Gasblasen, welche so klein sein können, daß sie im Mikrobereich liegen, beispielsweise einen Durchmesser von nur 40 Mikron haben. Die Blutabsaugvorrichtung besteht aus einer vertikal angeordneten zylindrischen Zyklonkammer, einem tangentialen Einlaß am oberen Kammerende, einem entgegengesetzt zur Zyklon-Rotationsbewegung tangential angeordneten Blutauslaß am unteren Kammerende, ein Entlüftungsrohr, welches in der Zyklon-Rotationsachse von oben nach unten bis unterhalb des Bluteinlasses in die Zyklonkammer hineinragt, einem zweiten Entlüftungsmittel in Form einer radialen Bohrung in einer oberen Verlängerung der Zyklonkammer oberhalb des Bluteinlasses, und einem zweiten Rohr, welches sich längs der Zyklon-Rotationsachse durch die gesamte Zyklonkammer und durch einen Teil des erstgenannten Rohres erstreckt und dazu dienen soll, daß an seiner Außenfläche Luftblasen sich sammeln und nach oben steigen können. Die DE-A-43 29 385 beschreibt, daß sie einen gegenüber der genannten GB-A-2 063 108 weiterentwickelten Luftabscheider betrifft, bei welchem Bluteinlaß und Blutauslaß an den voneinander angewandten Enden einer zylindrischen Wirbelkammer axial zueinander angeordnet sind. Der Bluteinlaß ist durch einen Leitschaufel-Körper gebildet und vor dem Blutauslaß befindet sich eine Filterkerze. Aufsteigende Blutbläschen gelangen in einen oberhalb des Leitschaufel-Körpers gelegenen Abschnitt der Wirbelkammer, wo ein Luftpolster gebildet ist, welches durch eine Bohrung entlüftet wird. Die DE-C-36 41 644 zeigt eine Blut-Durchflußkammer mit einem Bluteinlaß auf halber Kammerhöhe und einem in die Durchflußkammer eingetauchten Blutauslaßkanal. Im Blut enthaltene Luftblasen können nur durch die Auftriebskraft nach Archimedes nach oben steigen. Ferner zeigen die DE-C-36 24 363 und die US-A-5 451 321 Vorrichtungen mit mikroporösem Filtermaterial zum Filtern von Gasblasen oder anderen Blutverunreinigungen aus einem Blutstrom.

Durch die Erfindung soll die Aufgabe gelöst werden, bei der Absaugung von Blut von einem Patienten die vorgenannten Beeinträchtigungen des Blutes weitgehend zu reduzieren. Diese Aufgabe soll durch eine gewichtsmäßig leichte, preiswerte, und leicht bedienbare Vorrichtung gelöst werden.

Diese Aufgabe wird gemäß der Erfindung durch die Merkmale von Anspruch 1 gelöst.

Zweckmäßige Weiterbildungen der Erfindung sind Gegenstand der abhängigen Ansprüche.

Die Erfindung hat insbesondere folgende Vorteile: Die Blutabsaugvorrichtung kann nahe bei einem Patienten angeordnet werden; sie ist gewichtsmäßig sehr leicht; sie kann unter Verwendung handelsüblicher Kanülen preiswert hergestellt werden; ihre Handhabung ist einfach und für die Bedienungsperson nicht ermüdend; die Erfindung nützt zur Ausscheidung von Glasbläschen jeder Größe die Fliehkräfte einer Zyklonströmung, die Gasabsaug-Sogwirkung von oben auf das rotierende Blut, und die Auftriebskräfte nach Archimedes gleichzeitig und einander unterstützend aus, so daß bereits bei einem schwachen Sog einer Saugpumpe eine schonende und gleichzeitig wirksame Trennung der Gasphase aus der Blutphase stattfindet; wenn gemäß der Erfindung die Zyklonkammer die Form eines vom Bluteinlaß bis zum Blutauslaß nach unten hin enger werdenden Trichters hat, dann wird die zentrifugale Bewegungsenergie der Zyklonströmung vom Bluteinlaß bis zum Blutauslaß besser aufrechterhalten als bei einer nicht-trichterförmigen, zylindrischen Kammer, so daß auch bei kleinen Blutströmungsmengen und niedrigen Blutströmungsgeschwindigkeiten noch eine Blutrotation mit ausreichender Fliehkraft zum Ausscheiden von Luft aus dem Blut erzeugt wird, selbst bei einem schwachen Sog der Saugpumpe. Die Wirkungen und Vorteile der trichterförmigen Zyklonkammer werden weiter verbessert, wenn der Bluteinlaßkanal nicht nur ungefähr tangential, sondern auch schräg von oben nach unten in die Zyklonkammer gerichtet ist. In diesem Falle werden auch tropfenförmig kleine Blutmengen in der Zyklonkammer durch den Sog der Blut-Saugpumpe rotiert und von Luftbläschen befreit. Eine bessere Trennung von Blut und Gas aus dem Blut-Gas-Saugstrom wird gemäß einem besonderen Gedanken der Erfindung durch einen Gasraum über dem rotierenden Blutstrom erreicht, in welchem Gasblasen und Blut-Gas-Schaum genügend Zeit und Raum haben, um in einen Luftanteil und einen Blutanteil zu zerfallen, bevor das Gas mit Abstand oberhalb des rotierenden Blutstromes von einer Gas-Absaugvorrichtung abgesaugt wird.

Die Erfindung wird im folgenden mit Bezug auf die Zeichnungen anhand einer bevorzugten Ausführungsform als Beispiel beschrieben. In den Zeichnungen zeigen
- Fig. 1: eine Seitenansicht, teilweise längs der Ebene I-I von Fig. 3 geschnitten dargestellt, einer Blutabsaugvorrichtung gemäß der Erfindung im Maßstab 1:1,
- Fig. 2: eine schematische Längsschnitt-Ansicht längs der Ebene II-II von Fig. 1,
- Fig. 3: eine Draufsicht von oben auf die Blutabsaugvorrichtung von Fig. 1,
- Fig. 4: eine Seitenansicht, teilweise geschnitten, einer weiteren Ausführungsform nach der Erfindung,
- Fig. 5: eine Seitenansicht, teilweise geschnitten, einer nochmals weiteren Ausführungsform der Erfindung.

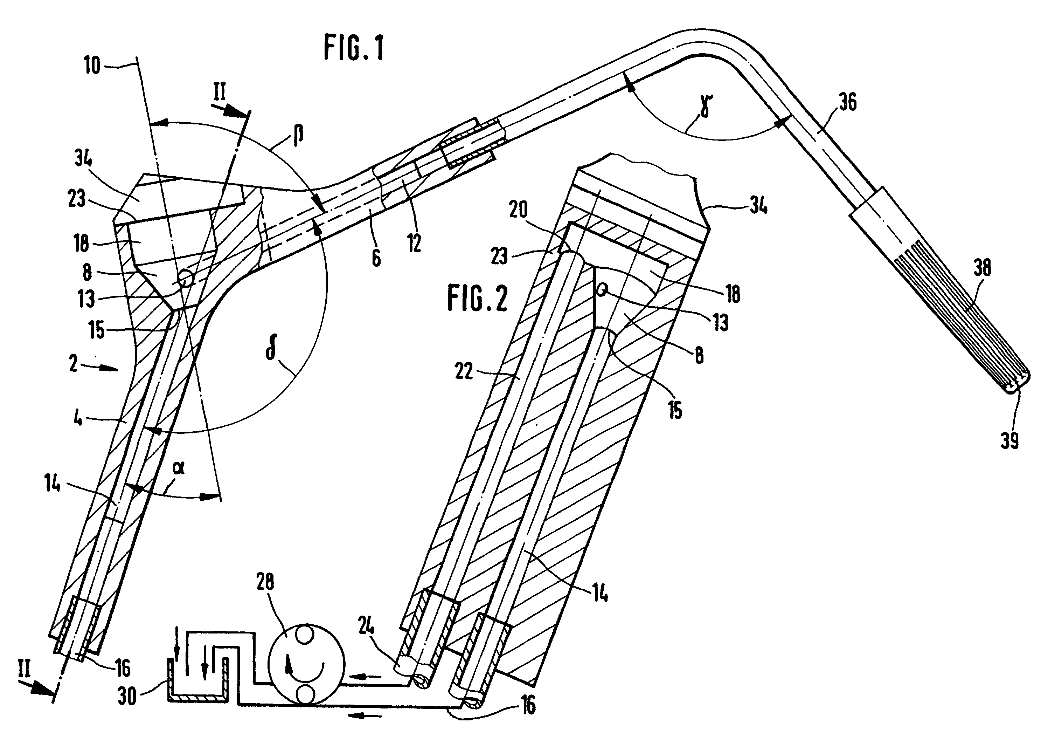

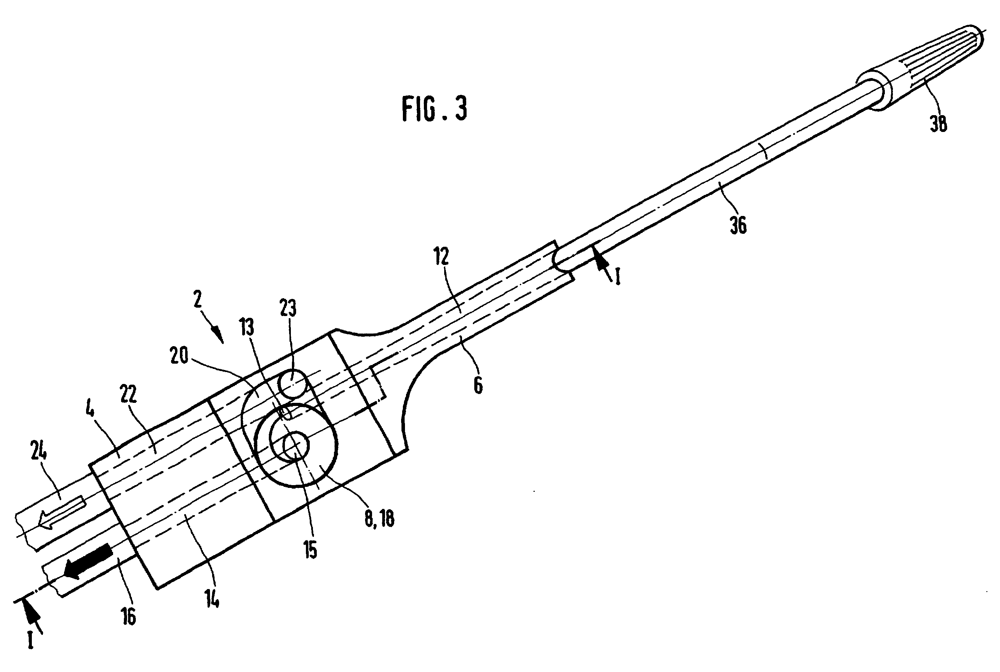

Die in den Fig. 1 bis 3 dargestellte Ausführungsform einer Blutabsaugvorrichtung nach der Erfindung zum Absaugen von Blut von einem Patienten, insbesondere von einer Wundstelle des Patienten, ist ein Ein-Hand-Gerät und enthält einen einteiligen oder mehrteiligen, pistolenförmigen Gerätekörper 2 aus Metall oder Kunststoff, der im wesentlichen aus einem Griff 4 und einem Lauf oder Schaft 6 besteht. Im Kreuzungsbereich zwischen Griff 4 und Schaft 6 ist im Gerätekörper 2 eine nicht-rotierende Zentrifugierkammer, im folgenden Zyklonkammer 8 genannt, zum Erzeugen eines mit gleichbleibender Drehrichtung zyklonartig rotierenden Blutstromes um eine aufrechte, vertikale oder nahezu vertikale Rotationsachse 10 gebildet. Die Zyklonkammer 8 hat von oben nach unten eine trichterartig enger werdende Form, damit ein durch sie hindurch gesaugter Blutstrom durch die ganze Zyklonkammer hindurch seine Bewegungsenergie im wesentlichen aufrechterhält, ohne daß eine starke Saugkraft in der Zyklonkammer erforderlich ist. Ein Bluteinlaß-Saugkanal 12 im Schaft 6 mündet bei einer Bluteinlaßöffnung 13 ungefähr tangential, und unter einem Winkel β von 90° oder vorzugsweise von weniger als 90° zur vertikalen Rotationsachse 10 schräg von oben nach unten, in den trichterförmigen oberen Abschnitt der trichterförmigen Zyklonkammer 8. Ein Blutauslaß-Saugkanal 14 erstreckt sich von einer Blutauslaßöffnung 15 am engen unteren Ende der Zyklonkammer 8 unter einem, nach unten offenen, Winkel α zwischen 0° und 90°, vorzugsweise ungefähr 30°, zur Rotationsachse 10 durch den Griff 4 zu einem an sein unteres Ende angeschlossenen Blutabsaugschlauch 16. Damit erstreckt sich der Blutauslaß-Saugkanal 14 entweder axial zur Rotationsachse 10 oder schräg von ihr weg von oben vorne nach unten hinten. Auf dem Strömungsweg zwischen dem Bluteinlaß-Saugkanal 12 und dem Blutauslaß-Saugkanal 14 rotiert der Blutstrom, ohne Umkehrströmungen, nur in einer einzigen Wirbel-Rotationsrichtung.

Die Zyklonkammer 8 ist durch einen über ihr angeordneten geschlossenen Gasraum 18 von ungefähr 5mm bis 15mm Höhe nach oben verlängert, welcher einen über die Zyklonkammer 8 seitlich hinausragenden Gasraumabschnitt 20 hat. Der Gasraum 18 dient als Speicherkammer zur temporären Aufnahme von Gasbläschen und Blutschaum, so daß sie Zeit und Raum zum Zerfallen in Luft und Blut haben. Dadurch wird der Blutanteil reduziert, der mit dem Gas, getrennt vom rotierenden Blutstrom abgesaugt wird. Der Gasraum 18 dient auch zur temporären Aufnahme von gegebenenfalls zeitweise aus der Zyklonkammer 8 aufsteigendem Blut. Der Querschnitt des Gasraumes 18 quer zur Rotationsachse 10 ist mindestens zweimal so groß wie der Querschnitt einer Gasauslaßöffnung 23.

Durch den Griff 4 erstreckt sich parallel zum Blutauslaß-Saugkanal 14 ein Gasauslaß-Saugkanal 22, der über die Gasauslaßöffnung 23 im Boden des seitlich überstehenden Gasraumabschnittes 20 mit dem Gasraum 18 verbunden ist. Die Gasauslaßöffnung 23 ist wesentlich höher angeordnet, z.B. 2mm - 10mm höher, als die Bluteinlaßöffnung 13. Am unteren Ende des Griffes 4 ist an den Gasauslaß-Saugkanal 22 ein Gasabsaugschlauch 24 angeschlossen. Der Gasabsaugschlauch 24 und der Blutabsaugschlauch 16 sind getrennt an eine Saugpumpe 28 angeschlossen, vorzugsweise an eine peristaltische Rollerpumpe, welche das abgesaugte Blut und die abgesaugte Luft auf getrennten Wegen in ein Blutreservoir 30 fördert. Dabei erzeugt die Saugpumpe 28 über den Blutauslaß-Saugkanal 14 und den Gasauslaß-Saugkanal 22 durch den Gasraum 18 und die Zyklonkammer 8 hindurch einen Saug-Unterdruck im Bluteinlaß-Saugkanal 12, so daß durch den Bluteinlaß-Saugkanal 12 Blut in die Zyklonkammer 8 gesaugt wird.

Der Gasraum 18 ist durch einen Deckel 34 geschlossen.

In das distale (vordere) Ende des Bluteinlaß-Saugkanals 6 ist eine Blutabsaug-Kanüle 36 lösbar gesteckt. Gemäß einer anderen Ausführungsform kann die Blutabsaug-Kanüle 36 auch ein einstückiges Teil mit dem Gerätekörper 2 bilden. Der vordere Endabschnitt 38 der Blutabsaugkanüle 36 ist um einen nach unten offenen Winkel γ von beispielsweise 105° nach unten abgewinkelt und mit Durchlaßöffnungen 39 versehen, um von einer Wundstelle eines Patienten Blut absaugen zu können, wenn eine Bedienungsperson den Gerätekörper 2 am Handgriff 4 in bequemer Handposition hält. Dementsprechend kann der Winkel γ der Blutabsaugkanüle 36 im Bereich zwischen 90° und 180° liegen.

Der Griff 4 und in ihm der Blutauslaß-Saugkanal 14 und der zu ihm parallele Gasauslaß-Saugkanal 22 erstrecken sich unter einem nach unten offenen Winkel α zwischen 0° und 90°, vorzugsweise ungefähr 30°, relativ zur Zyklon-Rotationsachse 10 schräg nach unten hinten. Die Winkel α und γ sind so aufeinander abgestimmt, daß die Vorrichtung bei der Blutabsaugung bequem am Griff getragen werden kann und dabei die Gasauslaßöffnung 23 immer höher angeordnet bleibt als die Bluteinlaßöffnung 13. Deshalb ist die Gasauslaßöffnung 23 vorzugsweise auf der von der Bluteinlaßöffnung 13 abgewandten Kammerseite angeordnet.

Der nach unten offene Winkel δ zwischen dem Blutauslaß-Saugkanal 14 und dem Gasauslaß-Saugkanal 22 einerseits und dem Bluteinlaß-Saugkanal 12 andererseits liegt, abhängig von den Winkeln α und β zwischen ungefähr 90° und 180°, vorzugsweise gemäß der dargestellten bevorzugten Ausführungsform bei 135°.

Gemäß der bevorzugten Ausführungsform haben der Gasauslaß-Saugkanal 22 und sein Gasabsaugschlauch 24 einen gleichen oder anderen Innenquerschnitt wie oder als der Blutabsaugkanal 14 und sein Blutabsaugschlauch 16, so daß Blut und Luft getrennt voneinander von der gleichen Saugpumpe 28 abgesaugt werden können, auch dann, wenn das Blut und die Luft verschiedene Strömungsvolumen haben.

Die von der Nähe der Bluteinlaßöffnung 13 bis zur Nähe der Blutauslaßöffnung 15 von oben nach unten enger werdende Konusform oder Trichterform der Zyklonkammer 8 gewährleistet, daß die Rotationsenergie des Blutstromes vom Bluteinlaß-Saugkanal 12 bis zum Blutauslaß-Saugkanal 14 ohne wesentliche Verlust aufrechterhalten wird, selbst wenn am Blutauslaß 15 nur ein geringer, das Blut schonender, Sog von der Saugpumpe 28 erzeugt wird.

Wenn der stromabwärtige Endabschnitt des Bluteinlaß-Saugkanals 12 bei der Bluteinlaßöffnung 13 nicht nur im wesentlichen tangential, sondern auch schräg von oben nach unten unter einem Winkel β von weniger als 90° in die Zyklonkammer 18 gerichtet ist, und damit in Richtung des Soges im Blutauslaß-Saugkanal 14 geneigt ist, dann werden von diesem Sog auch tropfenartig kleine Blutmengen in der Zyklonkammer 18 so schnell rotiert, daß Blut und Luft trenndende Fliehkräfte entstehen.

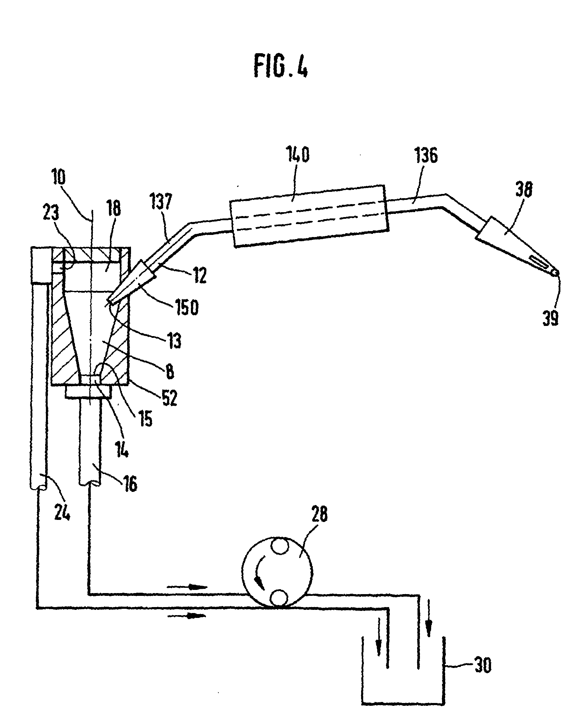

Bei der in Fig. 4 dargestellten Ausführungsform nach der Erfindung wird eine handelsübliche Blutabsaugkanüle 136 mit einem Handgriffteil 140 verwendet. Der stromabwärtige, hintere Endabschnitt 137 dieser Blutabsaugkanüle 136 bildet direkt oder durch ein Anschlußstück 150 den Bluteinlaß-Saugkanal 12 mit der Bluteinlaß-Öffnung 13 in der Zyklonkammer 8. Die Zyklonkammer 8 und der sich an ihr oberes Ende anschließende, nach oben erstreckende Gasraum 18 sind in einem Gehäuse 52 gebildet. Die weiteren in Fig. 4 gezeigten Details sind konstruktiv und zumindest funktionell identisch mit den Details der Fig. 1 bis 3 und sind mit gleichen Bezugszahlen versehen.

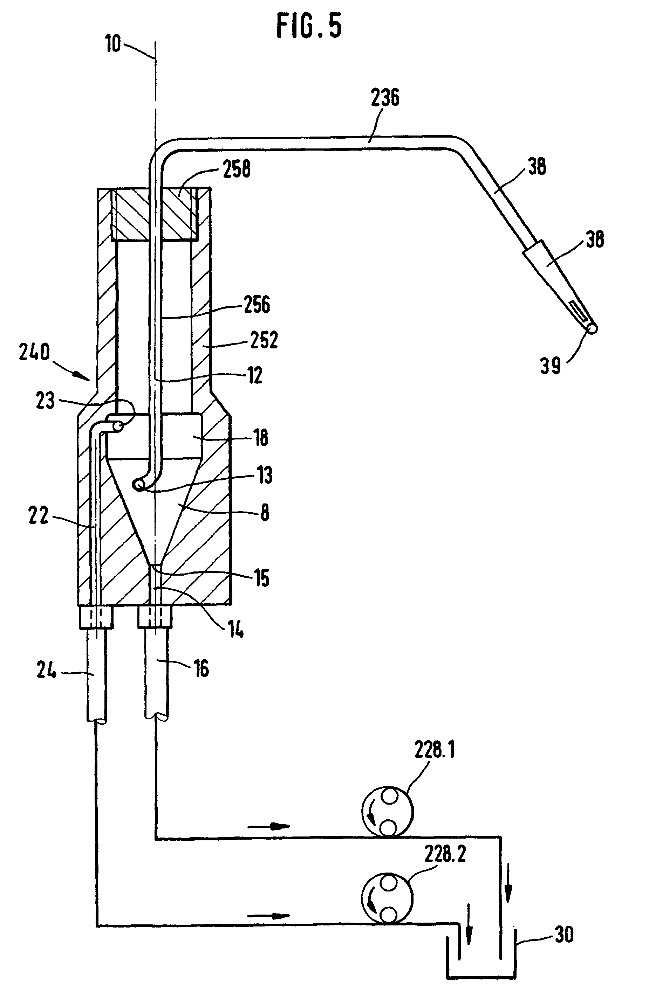

Bei der weiteren Ausführungsform der Erfindung gemäß Fig. 5 sind die Zyklonkammer 8 und der Gasraum 18 in einem Gehäuse 252 gebildet, welches gleichzeitig als Handgriffteil 240 ausgebildet ist, an welchem das gesamte Gerät mit einer Hand getragen werden kann. Die Blutabsaugkanüle 236 hat an ihrem stromabwärtigen, hinteren Ende ein vertikal von oben nach unten in das Gehäuse 252 hinein reichendes Rohrstück 256, welches den Bluteinlaß-Saugkanal 12 und, an seinem stromabwärtigen Ende, die Bluteinlaßöffnung 13 bildet. Der Gasraum 18 ist nach oben durch einen Verschluß 258 am oberen Ende des Gehäuses 252 verschlossen. Es ist eine Saugpumpe 228.1 für das Blut und eine Saugpumpe 228.2 für das Gas vorgesehen, jedoch können beide durch eine einzige Pumpe 28 entsprechend den Fig. 1 bis 4 ersetzt werden. Das Vakuum dieser Pumpen erzeugt durch die Gasauslaßöffnung 23, die Blutauslaßöffnung 15, die Zyklonkammer 18 und durch die Blutabsaugkanüle 236 hindurch einen Unterdruck oder Sog, durch welchen Blut von der Wundstelle eines Patienten in Öffnungen 39 am vorderen Katheterende eingesaugt wird. Die weiteren Details der Ausführungsform nach Fig. 5 sind gleich wie bei der Ausführungsform nach den Fig. 1 bis 3 und mit den gleichen Bezugszahlen versehen.

Bei den Fig. 4 und 5 fluchten die Blutauslaß-Saugkanäle 14 mit der vertikalen Rotationsachse 10 der als Zentrifugierkammer wirkenden Zyklonkammer 8.

"Gas" ist insbesondere Luft, kann jedoch auch ein anderes Medium sein.

## Patentansprüche

1. Blutabsaugvorrichtung zum Absaugen von Blut von einem Patienten, mit folgenden Merkmalen:
1.1. Die Blutabsaugvorrichtung ist als ein von Hand tragbares Gerät ausgebildet und mit einem Handgriffteil versehen;
1.2. das Gerät enthält eine nicht-rotierende Zentrifugierkammer (8) mit einem Bluteinlaß-Saugkanal (12) am oberen Kammerende im wesentlichen tangential zu einer im wesentlichen vertikalen Rotationsachse (10) und mit einem Blutauslaß-Saugkanal (14) am unteren Kammerende derart, daß vom Sog einer an den Blutauslaß-Saugkanal (14) anschließbaren Blut-Saugvorrichtung (28) ein Blutstrom mit gleichbleibender Rotationsrichtung um die Rotationsachse rotierend vom Bluteinlaß-Saugkanal (12) zum Blutauslaß-Saugkanal (14) gesaugt wird, wobei das Blut durch Fliehkräfte radial nach außen und in ihm enthaltenes Gas, insbesondere Luft, durch das Blut radial nach innen gedrängt werden;
1.3. die Zentrifugierkammer (8) ist oberhalb einer Bluteinlaßöffnung (13) des Bluteinlaß-Saugkanals (12) durch einen Gasraum (18) nach oben erweitert, welcher mit Höhenabstand oberhalb der Bluteinlaßöffnung (13) eine Gasauslaßöffnung (23) eines Gasauslaß-Saugkanals (22) hat, der an eine Gas-Saugvorrichtung (28) anschließbar ist, so daß Gasblasen und Blut-Gas-Schaum in diesen Gasraum (18) Zeit zum Zerfallen in Gasteile und Blutteile haben und dieses Gas sowie Gas aus dem rotierenden Blutstrom durch den Gasauslaß-Saugkanal (22) abgesaugt werden;
1.4. der Bluteinlaß-Saugkanal (12) ist mit einer Blutabsaugkanüle (36) verbunden oder durch deren hinteren Endabschnitt gebildet.

2. Blutabsaugvorrichtung nach Anspruch 1, wobei
die Zentrifugierkammer (8) eine von oben von der Nähe der Bluteinlaßöffnung (13) nach unten bis zur Nähe der Blutauslaßöffnung (15) trichterartig enger werdende Form hat.

3. Blutabsaugvorrichtung nach Anspruch 1 oder 2, wobei
der Bluteinlaß-Saugkanal (12), mindestens sein stromabwärtiger Endabschnitt an der Bluteinlaßöffnung (13), unter einem, nach oben öffnenden, Winkel von weniger als 90° zur Rotationsachse (10) schräg von oben nach unten in die Zentrifugierkammer (8) gerichtet ist.

4. Blutabsaugsvorrichtung nach einem der Ansprüche 1 bis 3, wobei
die Zentrifugierkammer (8) am vorderen oder oberen Ende des Handgriffteils (4) angeordnet ist.

5. Blutabsaugvorrichtung nach Anspruch 4, wobei
der Blutauslaß-Saugkanal (14) und der Gasauslaß-Saugkanal (22) sich nebeneinander durch den Handgriffteil in Grifflängsrichtung erstrecken.

6. Blutabsaugvorrichtung nach Anspruch 4 oder 5, wobei
der Handgriffteil (4) schräg zur Rotationsachse (10) und schräg zum Bluteinlaß-Saugkanal (12) angeordnet ist, wobei er sich von einem vorderen oberen Ende schräg nach hinten unten zu einem hinteren unteren Ende erstreckt.

7. Blutabsaugvorrichtung nach einem der Ansprüche 1 bis 3, wobei
der Handgriffteil (140) ein Teil der Blutabsaugkanüle (136) ist.

8. Blutabsaugvorrichtung nach Anspruch 7, wobei
die Zentrifugierkammer (8) an dem von der vorderen Kanülenspitze (38) abgewandten hinteren Ende (137) der Blutabsaugkanüle (136) angeordnet und der Handgriffteil (140) zwischen den beiden Kanülenenden angeordnet ist.

9. Blutabsaugvorrichtung nach einem der Ansprüche 1 bis 3, wobei
der Handgriffteil (4; 240) durch einen Körper gebildet ist, in welchem sich die Zentrifugierkammer (8) und der Gasraum (18) befinden.

10. Blutabsaugvorrichtung nach einem der vorhergehenden Ansprüche, wobei
der Blutauslaß-Saugkanal (14) eine andere Öffnungsquerschnitts-Größe hat als der Gasauslaß-Saugkanal (22).

11. Blutabsaugvorrichtung nach einem der vorhergehenden Ansprüche, wobei
eine Saugpumpe (28) mit zwei voneinander getrennten Saugwegen vorgesehen ist, welche die Blut-Saugvorrichtung und die Gas-Saugvorrichtung bildet.

## Claims

1. Blood extractor for extracting blood from a patient, with the following characteristics:
1.1 the blood extractor is designed as a device that can be carried by hand and provided with a handle portion;
1.2 the device contains a non-rotating centrifuging chamber (8) with a blood inlet suction channel (12) at the upper end of the chamber essentially tangentially to an essentially vertical axis of rotation (10) and with a blood outlet suction channel (14) at the lower end of the chamber such that, by the suction of a blood suction device (28) which can be connected to the blood outlet suction channel (14), a blood stream is drawn in rotating fashion with a constant direction of rotation from the blood inlet suction channel (12) to the blood outlet suction channel (14), wherein the blood is forced radially outwards by centrifugal forces, and gas contained in it, in particular air, is forced radially inwards by the blood;
1.3 the centrifuging chamber (8) is extended upwardly above a blood inlet opening (13) of the blood inlet suction channel (12) by a gas chamber (18) which at a vertical distance above the blood inlet opening (13) has a gas outlet opening (23) of a gas outlet suction channel (22) which can be connected to a gas suction device (28), so that gas bubbles and blood-gas foam in this gas chamber (18) have time to dissociate into gas portions and blood portions, and this gas as well as gas from the rotating blood stream are extracted through the gas outlet suction channel (22).
1.4 the blood inlet suction channel (12) is connected to a hollow blood extracting needle (36) or formed by the rear end section thereof.

2. Blood extractor according to claim 1, wherein the centrifuging chamber (8) has a shape narrowing like a funnel from the top in the vicinity of the blood inlet opening (13) to the bottom in the vicinity of the blood outlet opening (15).

3. Blood extractor according to claim 1 or 2, wherein the blood inlet suction channel (12), at least its downstream end section at the blood inlet opening (13), is directed obliquely from top to bottom into the centrifuging chamber (8) at an upwardly opening angle of less than 90° to the axis of rotation (10).

4. Blood extractor according to any of claims 1 to 3, wherein the centrifuging chamber (8) is arranged at the front or upper end of the handle portion (4).

5. Blood extractor according to claim 4, wherein the blood outlet suction channel (14) and the gas outlet suction channel (22) extend adjacent to each other through the handle portion in the longitudinal direction of the handle.

6. Blood extractor according to claim 4 or 5, wherein the handle portion (4) is arranged obliquely to the axis of rotation (10) and obliquely to the blood inlet suction channel (12), wherein it extends from a front upper end obliquely rearwards and downwards to a rear lower end.

7. Blood extractor according to any of claims 1 to 3, wherein the handle portion (140) forms part of the hollow blood extracting needle (136).

8. Blood extractor according to claim 7, wherein the centrifuging chamber (8) is arranged at the rear end (137) of the hollow blood extracting needle (136) facing away from the front tip (38) of the hollow needle, and the handle portion (140) is arranged between the two ends of the hollow needle.

9. Blood extractor according to any of claims 1 to 3, wherein the handle portion ( 4; 240) is formed by a body in which are located the centrifuging chamber (8) and the gas chamber (18).

10. Blood extractor according to any of the preceding claims, wherein the blood outlet suction channel (14) has a different opening cross-section size to the gas outlet suction channel (22).

11. Blood extractor according to any of the preceding claims, wherein a suction pump (28) with two separate suction paths is provided, which forms the blood suction device and the gas suction device.

## Revendications

1. Dispositif d'aspiration de sang pour aspirer du sang d'un patient, avec les caractéristiques suivantes :
1.1 le dispositif d'aspiration de sang est réalisé sous forme d'appareil portable à la main et est pourvu d'une partie formant poignée ;
1.2 l'appareil contient une chambre de centrifugation non rotative (8), avec un canal d'aspiration (12) formant admission de sang à l'extrémité supérieure de la chambre, essentiellement tangentiellement à un axe de rotation (10) essentiellement vertical, et avec un canal d'aspiration (14) formant évacuation de sang à l'extrémité inférieure de la chambre, de telle sorte que, par l'aspiration d'un dispositif aspirateur de sang (28) pouvant être raccordé au canal d'aspiration (14) formant évacuation de sang, un flux de sang est, en tournant autour de l'axe de rotation avec une direction de rotation constante, aspiré du canal d'aspiration (12) formant admission de sang vers le canal d'aspiration (14) formant évacuation de sang, le sang étant refoulé radialement vers l'extérieur par les forces centrifuges, et le gaz contenu dans le sang, notamment l'air, étant refoulé radialement vers l'intérieur par le sang ;
1.3 la chambre de centrifugation (8) est, au-dessus d'une ouverture d'admission de sang (13) du canal d'aspiration (12) formant admission de sang, élargie vers le haut par un espace pour gaz (18) qui possède, à distance en hauteur au-dessus de l'ouverture d'admission de sang (13), une ouverture d'évacuation de gaz (23) d'un canal d'aspiration (22) formant évacuation de gaz qui peut être raccordé à un dispositif aspirateur de gaz (28), de sorte que des bulles de gaz et de l'écume de sang et de gaz présents dans cet espace pour gaz (18) ont le temps de se décomposer en particules de gaz et particules de sang, et ce gaz ainsi que le gaz provenant du flux de sang rotatif sont aspirés par le canal d'aspiration (22) formant évacuation de gaz ;
1.4 le canal d'aspiration (12) formant admission de sang est relié à une canule d'aspiration de sang (36) ou est formé par la partie terminale arrière de celle-ci.

2. Dispositif d'aspiration de sang selon la revendication 1, dans lequel la chambre de centrifugation (8) possède une forme allant en se rétrécissant en entonnoir de haut, à proximité de l'ouverture d'admission de sang (13), en bas, à proximité de l'ouverture d'évacuation de sang (15).

3. Dispositif d'aspiration de sang selon la revendication 1 ou 2, dans lequel le canal d'aspiration (12) formant admission de sang, à savoir au moins sa partie terminale située en aval dans la direction d'écoulement, au niveau de l'ouverture d'admission de sang (13), est dirigé en oblique de haut en bas par rapport à l'axe de rotation (10) dans la chambre de centrifugation (8), sous un angle inférieur à 90°, s'ouvrant vers le haut.

4. Dispositif d'aspiration de sang selon l'une des revendications 1 à 3, dans lequel la chambre de centrifugation (8) est disposée à l'extrémité avant ou supérieure de la partie formant poignée (4).

5. Dispositif d'aspiration de sang selon la revendication 4, dans lequel le canal d'aspiration (14) formant évacuation de sang et le canal d'aspiration (22) formant évacuation de gaz s'étendent l'un à côté de l'autre à travers la partie formant poignée, dans la direction longitudinale de la poignée.

6. Dispositif d'aspiration de sang selon la revendication 4 ou 5, dans lequel la partie formant poignée (4) est disposée en oblique par rapport à l'axe de rotation (10) et en oblique par rapport au canal d'aspiration (12) formant admission de sang, en s'étendant, depuis une extrémité supérieure avant, en oblique vers l'arrière et le bas jusqu'à une extrémité inférieure arrière.

7. Dispositif d'aspiration de sang selon l'une des revendications 1 à 3, dans lequel la partie formant poignée (140) est une partie de la canule d'aspiration de sang (136).

8. Dispositif d'aspiration de sang selon la revendication 7, dans lequel la chambre de centrifugation (8) est disposée à l'extrémité arrière (137), opposée à la l'extrémité avant de canule (38), de la canule d'aspiration de sang (136), et la partie formant poignée (140) est disposée entre les deux extrémités de la canule.

9. Dispositif d'aspiration de sang selon l'une des revendications 1 à 3, dans lequel la partie formant poignée (4 ; 240) est formée par un corps dans lequel se trouvent la chambre de centrifugation (8) et l'espace pour gaz (18).

10. Dispositif d'aspiration de sang selon l'une des revendications précédentes, dans lequel le canal d'aspiration (14) formant évacuation de sang possède une taille de section d'ouverture différente de celle du canal d'aspiration (22) formant évacuation de gaz.

11. Dispositif d'aspiration de sang selon l'une des revendications précédentes, dans lequel il est prévu une pompe aspirante (28) dotée de deux chemins d'aspiration séparés l'un de l'autre, pompe qui constitue le dispositif aspirateur de sang et le dispositif aspirateur de gaz.
